# FASCICULE DE BREVET EUROPEEN

(11) **EP 1 304 969 B1**
(45) Date de publication et mention de la délivrance du brevet: **02.11.2005**
(21) Numéro de dépôt: 01960855.3
(22) Date de dépôt: 01.08.2001
(51) Int. Cl.: A61B 17/80

(54) **PLAQUE D'OSTEOSYNTHESE**
KNOCHENPLATTE
OSTEOSYNTEHSIS PLATE

(30) Priorité: 02.08.2000 FR 0010188
(43) Date de publication de la demande: 02.05.2003
(73) Titulaire: Caron, Philippe, Papeete 98713, BP 1040 Tahiti (FR)
(72) Inventeur: Caron, Philippe, Papeete 98713, BP 1040 Tahiti (FR)
(74) Mandataire: Cardy, Sophie Marie
(86) Numéro de dépôt international: PCT/FR2001/002511
(87) Numéro de publication internationale: WO 2002/009607

(56) Documents cités:
- WO-A-99/23964
- US-A- 4 905 679

## Description

La présente invention concerne une plaque d'ostéosynthèse permettant d'effectuer une réduction de fracture osseuse, à savoir de remettre et de maintenir en position deux fragments osseux l'un par rapport à l'autre.

De telles plaques d'ostéosynthèse sont classiquement notamment utilisées au cours d'interventions de chirurgie maxillo-faciale, que ce soit à visée réparatrice pour réduire une fracture accidentelle, ou à visée fonctionnelle et/ou esthétique.

Les ostéotomies de la mandibule et du maxillaire rentrent dans cette dernière catégorie et doivent permettre, à la suite d'une préparation orthodontique quasi systématique, de modifier l'emplacement du maxillaire inférieur (mandibule) et/ou du maxillaire supérieur en vue de positionner les deux arcades dentaires de manière congruente, et ceci afin d'obtenir une occlusion la plus satisfaisante possible.

Au cours de ces ostéotomies, le chirurgien réalise un trait d'ostéotomie permettant de séparer en deux fragments osseux le maxillaire supérieur et/ou le maxillaire inférieur. Ainsi, dans le cas d'une ostéotomie de la mandibule, c'est le corps mandibulaire, formant le support des dents, qui est séparé, à droite et à gauche, du ramus mandibulaire correspondant. L'extrémité supérieure du ramus mandibulaire (condyle) coopère avec une cavité articulaire de l'os temporal, dénommée cavité glénoïde ou glène, pour former l'articulation temporo-mandibulaire permettant au maxillaire inférieur d'être mobile. Dans le cas de l'ostéotomie du maxillaire supérieur, c'est tout le plateau maxillaire qui est séparé du crâne par une ligne d'ostéotomie qui réalise une disjonction crano-faciale.

Après désolidarisation des deux fragments osseux du maxillaire concerné, le fragment libre est déplacé dans une ou plusieurs directions puis positionné correctement avant d'être bloqué.

Il est connu de réaliser ce blocage entre les deux fragments osseux, c'est-à-dire l'ostéosynthèse, au moyen soit de fils d'acier, soit de plaques fixées par des vis, soit de vis bicorticales.

Une ostéosynthèse aux fils d'acier entraîne un blocage intermaxillaire post-opératoire de l'ordre de trois à quatre semaines. Ce blocage est nécessaire compte tenu de la faible résistance mécanique des fils d'acier et de leur impossibilité à immobiliser à eux seuls complètement le foyer de fracture engendré par l'ostéotomie.

Ce blocage bi-maxillaire prolongé entraîne des suites opératoires relativement inconfortables pour le patient.

Afin notamment d'améliorer le confort du patient, a été développée l'ostéosynthèse rigide par plaques ou par vis bicorticales. Ces deux techniques permettent de limiter très fortement le temps de blocage, voire de l'annuler au moyen d'un blocage élastique. Toutefois, cette technique d'ostéosynthèse rigide empêche, contrairement à l'ostéosynthèse aux fils d'acier, tout déplacement ultérieur des deux fragments osseux après le positionnement et le blocage. Une telle mobilité après blocage permet en effet au condyle de retrouver sa position initiale après l'ostéosynthèse, c'est-à-dire sa position physiologique au sein de l'articulation temporo-mandibulaire.

En conséquence, on comprend que l'ostéosynthèse rigide impose au chirurgien un positionnement précis et sûr des plaques, ce positionnement étant définitif, c'est-à-dire qu'il ne peut pas être modifié ultérieurement.

Si l'on tient compte de tous les paramètres que le chirurgien doit prendre en compte pour effectuer le positionnement précité, au nombre desquels on doit compter notamment la meilleure congruence possible entre les arcades dentaires ainsi que le maintien d'une bonne fonction de l'articulation temporo-mandibulaire, alors que celle-ci n'est pas visible, on comprend que le positionnement correct des plaques est délicat, voire aléatoire.

Cette difficulté de positionnement des plaques lors des interventions d'ostéotomie entraîne souvent, pour le chirurgien, la nécessité de recommencer la pose des plaques d'ostéosynthèse. La pose de nouvelles plaques doit parfois être réalisée plusieurs fois avant d'aboutir à une position relative satisfaisante entre les deux fragments osseux.

En outre, le nombre des emplacements possibles pour les plaques d'ostéosynthèse est limité du fait de la faible surface osseuse disponible pour positionner ces plaques et réaliser les forages nécessaires aux vis de fixation, chaque nouvelle pose de plaque imposant de réaliser de nouveaux forages. Il convient également de préciser que l'accès à l'emplacement où est effectué le trait d'ostéotomie puis où est posée chaque plaque reste difficile tout au long de l'intervention.

La présente invention a pour objectif de résoudre les problèmes précités inhérents aux plaques d'ostéosynthèse rigide afin de permettre, après la fixation de la plaque d'ostéosynthèse, un réglage ultérieur possible de la position relative des deux fragments osseux qui sont à réunir mécaniquement.

On connaît du document US-A-4905679 des plaques avec des branches de réglage susceptibles d'être déformées. Le préambule de la revendication 1 est fondé sur ce document.

Selon l'invention, on propose une plaque d'ostéosynthèse qui est munie de quatre branches reliées entre elles deux à deux au niveau de quatre sommets percés chacun par un alésage destiné à coopérer avec une vis de fixation, caractérisée en ce que la largeur d'au moins l'une des branches est inférieure au diamètre desdits alésages afin de constituer au moins une branche de réglage susceptible d'être déformée de manière à modifier l'écart entre les deux sommets situé aux extrémités de ladite branche de réglage.

On comprend que la plaque selon l'invention, comporte au moins une branche de réglage constituée par une fine barre métallique susceptible d'être tordue au moyen d'une pince afin de réaliser une déformation plastique. En modifiant l'écart entre tes deux points de fixation constitués par les sommets situés aux deux extrémités de cette barre fine, cette déformation plastique modifie la géométrie de la plaque d'ostéosynthèse et donc le positionnement relatif entre les deux fragments osseux sur lesquels cette plaque a été montée.

Une telle plaque, réalisée classiquement en alliage à base de titane ou éventuellement en alliage chrome-cobalt-molybdène, doit répondre aux critères de résistance mécanique imposés par leur emplacement. En effet, dans le cas d'une ostéotomie mandibulaire, il convient de rappeler que la réduction de fracture, engendrée par l'ostéotomie, doit résister à la force exercée par les muscles masticateurs qui sont des muscles puissants exerçant une force comparable à celle exercée par certains autres muscles du squelette.

Toutefois, afin de permettre le réglage de la position relative entre les fragments osseux, au moyen d'une torsion de la branche de réglage, cette dernière doit pouvoir être déformée sans remettre en cause la solidité de la fixation de la plaque d'ostéosynthèse par les vis disposées au niveau des quatre sommets de la plaque et qui sont vissées dans les fragments osseux.

A cet effet, selon une forme préferée de la présente invention, il est prévu que la largeur de ladite branche de réglage soit comprise entre 20 % et 60 %, de préférence entre 35 et 45 % du diamètre des alésages des sommets.

Selon une solution préférentielle, il est prévu que la largeur de la branche de réglage est de l'ordre de 40 % du diamètre des alésages.

La plaque d'ostéosynthèse présente la forme d'un parallélogramme les quatre branches étant parallèles deux à deux. Ce qui est avantageux pour les interventions de chirurgie particulières que sont les ostéotomies mandibulaires.

Selon une configuration avantageuse, la plaque d'ostéosynthèse comporte deux branches longues et deux branches courtes, les extrémités des branches longues étant davantage éloignées l'une de l'autre que les extrémités des branches courtes et au moins l'une des deux branches longues présente une largeur inférieure au diamètre desdits alésages afin de constituer ladite branche de réglage.

De préférence les deux branches longues présentent une largeur inférieure au diamètre desdits alésages afin de constituer deux branches de réglage.

Le parallélogramme présente un angle au sommet aigu compris entre 50° et 80°, de préférence sensiblement égal à 65°.

En outre, de manière préférentielle, les alésages de la plaque d'ostéosynthèse présentent une fraisure sur les deux faces de la plaque. Cette disposition permet de réaliser des plaques réversibles utilisables à souhait, à droite ou à gauche.

Selon une autre disposition préférentielle, l'épaisseur de la plaque d'ostéosynthèse est sensiblement égale à la largeur de ladite branche de réglage.

L'invention sera mieux comprise, et les caractéristiques secondaires et leurs avantages apparaîtront au cours de la description d'un mode de réalisation donnée ci-dessous à titre d'exemple.

Il est entendu que la description et les dessins ne sont donnés qu'à titre indicatif et non limitatif.

Il sera fait référence aux dessins annexés, dans lesquels :
- la figure 1 est une vue de gauche partielle des os de la tête, après réalisation de la ligne d'ostéotomie au cours d'une ostéotomie sagittale de la mandibule,
- la figure 2 est une vue similaire à la figure 1 avec les deux fragments osseux de la mandibule remis en place l'un contre l'autre dans leur position initiale avec une plaque d'ostéosynthèse selon l'invention,
- la figure 3 représente une plaque d'ostéosynthèse selon l'invention en élévation,
- la figure 4 est une vue en coupe selon la direction IV-IV de la figure 3,
- la figure 5 est une vue similaire à la figure 4 présentant une variante de réalisation de la plaque d'ostéosynthèse selon l'invention,
- la figure 6 représente l'action de réglage par déformation de la plaque d'ostéosynthèse selon la présente invention,
- la figure 7 représente les mouvements relatifs engendrés par l'action réalisée sur la figure 6, et
- la figure 8 est une vue similaire à celle de la figure 7 dans le cas où l'on réalise une déformation de la plaque d'ostéosynthèse dans le sens inverse par rapport à celle de la figure 6.

Dans la suite de la description, la plaque d'ostéosynthèse selon la présente invention est illustrée dans son utilisation à la réalisation d'une ostéotomie de la mandibule mais il convient de ne pas limiter les applications possibles de la plaque d'ostéosynthèse selon la présente invention à ce type d'intervention, ni aux interventions de chirurgie maxillo-faciale.

Toutefois, il convient de remarquer, compte tenu du faible encombrement de la plaque d'ostéosynthèse selon la présente invention, que celle-ci convient tout particulièrement aux opérations de chirurgie maxillo-faciale qui impliquent des os dont la mobilité, les dimensions géométriques et les forces exercées sur eux, obligent à l'utilisation de systèmes de fixation de faibles dimensions.

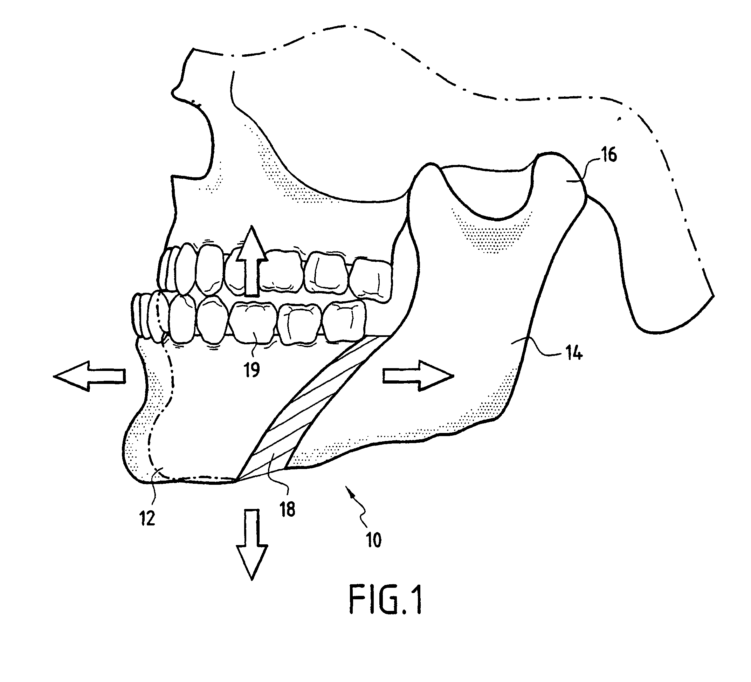

Si l'on se reporte à la figure 1, est visible une mandibule humaine 10 composée d'un corps mandibulaire 12 et d'un ramus mandibulaire 14. Le corps mandibulaire 12 et le ramus mandibulaire 14 sont séparés l'un de l'autre au niveau d'un trait d'ostéotomie 18 linéaire, oblique de haut en bas et qui longe la ligne oblique externe en la prolongeant le plus loin possible en bas et en avant jusqu'à la vertical de la première molaire 19.

Après réalisation de cette ostéotomie de chaque côté de la mandibule 10, le chirurgien peut alors déplacer le corps mandibulaire 12 par rapport au ramus mandibulaire 14 comme indiqué par les quatre flèches épaisses de la figure 1. Ce mouvement relatif va permettre d'arriver dans une position intermédiaire correspondant à une position en hyper correction par rapport à la position définitive visée par le chirurgien. C'est dans cette position intermédiaire (illustrée en traits pleins sur la figure 1) que devront être bloqués les deux fragments osseux 12 et 14 l'un par rapport à l'autre.

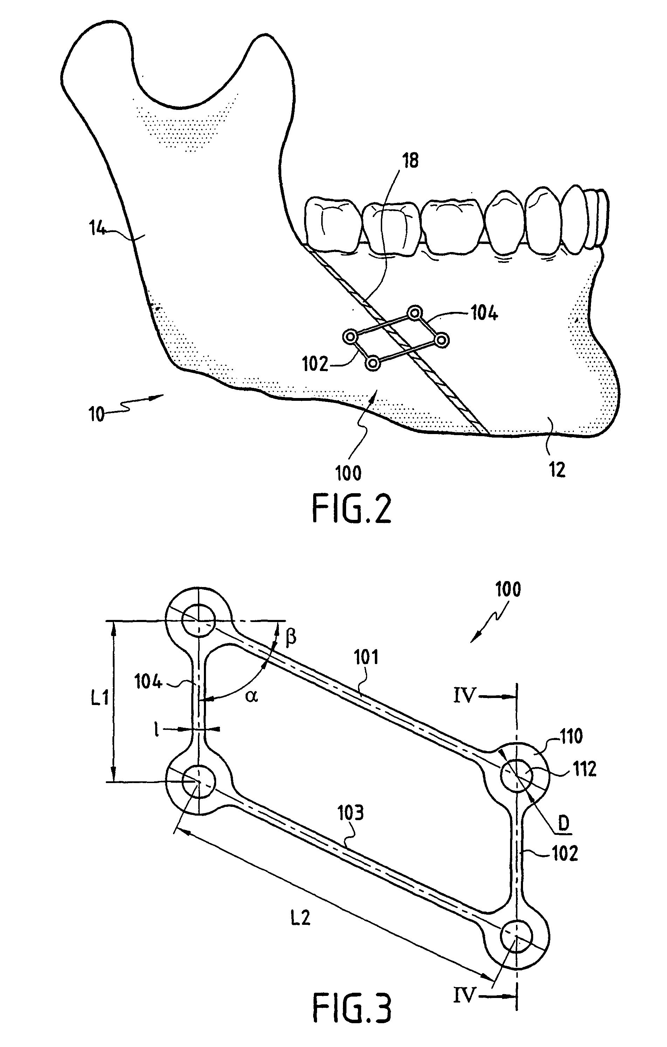

Pour effectuer ce blocage, comme on peut le voir sur la figure 2, une plaque d'ostéosynthèse 100 selon l'invention est utilisée, cette plaque étant montée à cheval de part et d'autre de la ligne oblique 18 avec deux points d'ancrage possibles sur chacun des deux fragments osseux 12 et 14.

Comme illustré sur la figure 3, la plaque d'ostéosynthèse 100 forme un quadrilatère à quatre branches rectilignes 101 à 104 reliées les unes aux autres, deux par deux, au niveau d'un sommet 110 présentant un alésage 112 qui est destiné à recevoir la tête d'une vis dont la tige filetée va pénétrer dans l'os.

Selon une caractéristique essentielle de la présente invention, afin de permettre le réglage précis et fin de la position de la plaque d'ostéosynthèse 100 sur les deux fragments osseux 12 et 14, l'une au moins des branches de la plaque d'ostéosynthèse 100, destinée à chevaucher le trait d'ostéotomie, est susceptible d'être déformée plastiquement.

Sur le mode de réalisation illustré sur la figure 3, les quatre branches 101 à 104 sont disposées parallèlement l'une à l'autre deux à deux et constituent un parallélogramme formé de deux branches longues 101 et 103 et de deux branches courtes 102 et 104 qui présentent toutes une largeur I identique, faible, permettant de déformer plastiquement ces branches 101 à 104 de manière relativement facile.

En pratique, la largeur I des branches 101 et 104 doit être choisie de façon à pouvoir être déformée au moyen d'une pince tout en pouvant résister aux contraintes exercées par les muscles masticateurs entre les deux fragments osseux 12 et 14, d'une part, et aux contraintes externes exercées sur la mandibule 10 notamment lors de la mastication, d'autre part.

Afin d'atteindre cet objectif, la largeur I est inférieure au diamètre D des alésages 112 de chacun des sommets 110. Ainsi, on choisira avantageusement une largeur I comprise entre 20 % et 60 % du diamètre D des alésages 112, cette largeur I étant de préférence comprise entre 35 et 45 % du diamètre D des alésages 112.

Dans le mode de réalisation préférentiel illustré sur la figure 3, la largeur I des branches 101 à 104 est égale à environ 40 % du diamètre D des alésages 112.

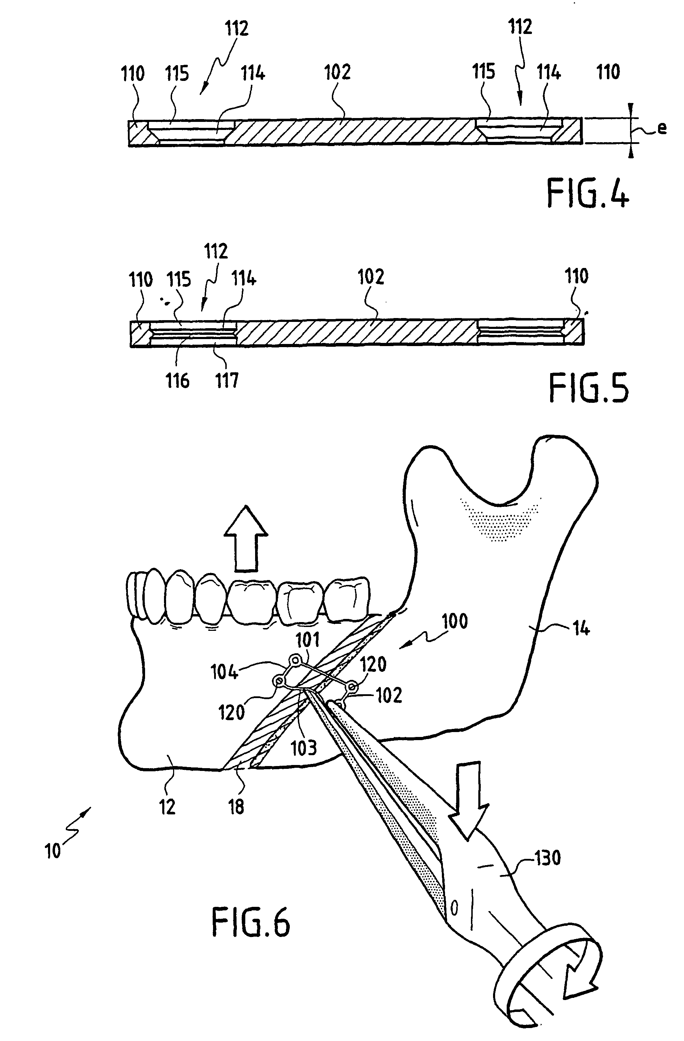

Comme on peut le voir sur la figure 4 présentant en coupe la branche courte 102, les alésages 112 comportent, pour le logement de la tête de vis, une fraisure 114 surmontée d'un lamage 115.

Afin de pouvoir utiliser la plaque d'ostéosynthèse 100 en forme de parallélogramme sur l'une ou l'autre de ses faces, il est avantageusement prévu, comme illustré sur la figure 5, que chaque alésage 112 puisse comprendre deux fraisures 114, 116 inversées, surmontées chacune d'un lamage 115, 117. Cette possibilité avantageuse sera mieux comprise au vu des explications qui suivent concernant le montage de la plaque d'ostéosynthèse sur la mandibule et sur le réglage que cette plaque permet de réaliser.

Comme on peut le voir sur la figure 2, la plaque 100 est montée sur la mandibule 10, à cheval sur la ligne d'ostéotomie 18, les branches courtes 102 et 104 étant disposées parallèlement à cette ligne d'ostéotomie 18 de part et d'autre de cette ligne, respectivement sur le corps de mandibule 12 et sur le ramus mandibulaire 14. Des vis de fixation 120 sont montées dans l'un au moins des alésages 112 de chaque branche courte 102, 104 pour fixer la plaque 100 à la fois au corps mandibulaire 12 et au ramus mandibulaire 14.

Une pince 130, dont les branches passent de part et d'autre de l'une des deux branches longues 101, 103, permet de déformer cette branche longue 101 ou 103 de la plaque d'ostéosynthèse 100. En effet, comme on peut le voir sur la figure 6, les extrémités des branches de la pince 130 sont insérées dans le trait d'ostéotomie 18 et enserrent la branche longue inférieure 103 en étant écartées de la largeur I à l'emplacement de cette branche longue 103 qui forme la branche de réglage.

Avant la fixation de la plaque d'ostéosynthèse 100 comme indiqué sur la figure 2, le plan de cette plaque est cintré afin que la plaque 100 puisse épouser le profil courbe de la face externe de la mandibule 10.

Sur la figure 6, la pince 130 exerce une rotation dans le sens horaire au niveau de la partie centrale de la branche longue inférieure 103, ce qui crée un déplacement vertical relatif (voir les flèches épaisses) entre les fragments osseux 12 et 14.

Ce déplacement vertical est rendu définitif ultérieurement à la déformation de la branche longue 103 comme il sera expliqué plus loin. Un tel déplacement relatif est possible car la plaque ainsi déformée conserve des branches courtes 102 et 104 parallèles l'une à l'autre, ce qui permet de faire glisser les fragments osseux 12 et 14 l'un par rapport à l'autre parallèlement à la ligne d'ostéotomie 18.

Cette procédure permet de conserver une surface de contact osseuse importante entre les fragments osseux 12 et 14, ce qui entraînera une récupération plus facile et plus rapide.

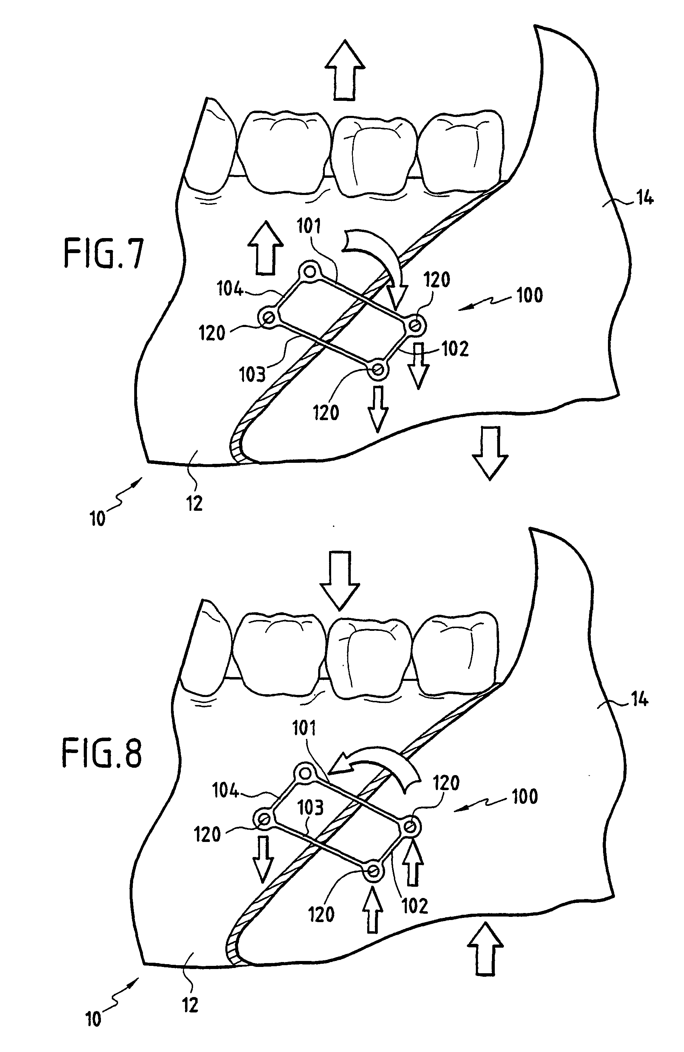

Selon une procédure opératoire préférentielle telle qu'illustrée sur la figure 7, deux vis 120 sont disposées dans les deux alésages 112 de la branche courte 102 destinée à être fixée sur le ramus mandibulaire 14 tandis qu'une seule vis 120 est disposée dans l'alésage 112 correspondant à la partie inférieure de la branche courte 104 et qui est fixée sur le corpus mandibulaire 12. Cette vis unique qui est présente au niveau du corpus mandibulaire 12 forme un axe de rotation qui facilite le déplacement vertical du corpus mandibulaire 12 par rapport au ramus mandibulaire 14 lors de la déformation de la branche de réglage formée de la branche longue inférieure 103.

Sur la figure 8, le même montage initial de la plaque 100 étant réalisé, sont représentés les mouvements relatifs engendrés par une torsion dans le sens anti-horaire de la branche longue 103, ce qui a pour conséquence d'abaisser le corpus mandibulaire 12 par rapport au ramus mandibulaire 14.

Lors de la réalisation de cette déformation, le chirurgien parvient ainsi à ajuster le positionnement relatif entre le corpus mandibulaire 12 et le ramus mandibulaire 14 pour obtenir, entre le maxillaire inférieur et le maxillaire supérieur, une bonne congruence des arcades dentaires tout en conservant une bonne articulation temporo-mandibulaire.

Ainsi, on comprend que la déformation de l'une des branches longues 101 ou 103 (ou la déformation de ces deux branches longues 101 et 103) peut être réalisée progressivement en plusieurs étapes jusqu'à parvenir à la position correcte.

De cette manière, on évite, contrairement à l'art antérieur, de devoir nécessairement fixer définitivement la plaque d'ostéosynthèse dans sa position correcte et donc d'avoir, éventuellement, à retirer puis à placer successivement l'une après l'autre plusieurs plaques jusqu'à parvenir à la position idéale tant par la congruence des arcades dentaires que par une bonne articulation temporo-mandibulaire.

Quand cette position finale est atteinte par l'ajustage de la position relative entre les deux fragments osseux 12, 14, qui est rendue possible par la déformation précitée de la plaque d'ostéosynthèse 100 selon l'invention, l'ostéosynthèse est verrouillée par la mise en place d'une quatrième vis dans l'alésage de la branche longue supérieure 101 située en regard du corpus mandibulaire 12. Après ce verrouillage, aucun mouvement relatif n'est plus possible entre les deux fragments osseux 12, 14 qui resteront dans la position finale précitée.

La plaque d'ostéosynthèse 100 présentant une forme de parallélogramme, le réglage s'effectue par contraction d'au moins une des deux branches longues 101, 103 qui forment deux branches de réglage. Cette contraction est très simple à réaliser par simple torsion exercée sur l'une ou l'autre (ou les deux) branche(s) de réglage.

En pratique, la première étape de fixation de la plaque 100 par les trois premières vis est réalisée en disposant le corpus mandibulaire 12 en hyper correction (illustration en traits pleins sur la figure1) par rapport au maxillaire supérieur de façon à pouvoir effectuer le réglage précis par la déformation de l'une au moins des deux branches longues 101, 103 de la plaque d'ostéosynthèse 100.

Outre le déplacement vertical relatif entre les deux fragments osseux 12, 14, la plaque 100 selon l'invention permet également de réaliser éventuellement un mouvement relatif transversal entre le corpus mandibulaire 12 et le ramus mandibulaire 14, c'est-à-dire un déplacement relatif entre les arcades dentaires dans le sens vestibulo-lingual. Un tel mouvement s'obtient lors de la déformation par contraction précitée de l'une au moins des branches longues 101, 103 si, préalablement à la pose de la plaque 100, celle-ci a subi un cintrage concave ou convexe qui s'ajoute au cintrage correspondant à la forme naturelle de la face externe de la mandibule 10.

Pour l'usage en ostéotomie mandibulaire telle que décrite précédemment, la plaque d'ostéosynthèse 100, selon la présente invention, présentera de préférence les dimensions suivantes en regard de la figure 3 : I = 0,8 mm, cette largeur étant utilisée pour les quatre branches rectilignes 101 à 104 de la plaque 100, le diamètre D des alésages 112 étant égal à 2 mm, chaque sommet 110 de la plaque 100 présente une forme de disque de diamètre égal à 4 mm de sorte que chaque sommet 110 forme un anneau ayant une largeur de matière égale à 1 mm, soit une largeur de matière plus importante que la largeur I de chacune des branches 101 à 104.

En outre, ramenée au centre des alésages 112, la longueur L1 des branches courtes 102 et 104 est égale à 10 mm, tandis que la longueur L2 des branches longues 101 et 103 (entre le centre des deux alésages 112) peut varier entre 15 et 25 mm selon la taille de la mandibule du patient à opérer.

Concernant l'épaisseur e de la plaque 100, celle-ci peut varier entre 0,6 et 1,4 mm et sera de préférence de l'ordre de grandeur de la largeur l des branches de réglage. Dans le cas de la plaque 100 illustrée sur les figures 3 à 5, e=1 mm.

D'après les explications qui précèdent, on comprend que la réalisation d'une ostéotomie mandibulaire nécessite l'emploi de deux plaques d'ostéosynthèse 100, une plaque pour le côté droit (figures 6 à 8) et une plaque symétrique pour le côté gauche (figures 2 et 3). En effet, comme on le voit sur la figure 4, une seule face de la plaque 100 comporte les fraisures 114 pour le logement de la tête des vis.

Ainsi, la variante de réalisation préférentielle selon laquelle chaque alésage 112 comprend deux fraisures 114, 116 situées chacune sur une face différente de la plaque 100 (voir la figure 5 qui représente cette variante de réalisation) permet de réaliser un seul modèle de plaque qui se monte indépendamment à droite ou à gauche selon la face de la plaque qui se trouve en contact sur la mandibule du patient.

Afin de faciliter le mouvement relatif entre les deux fragments osseux 12 et 14 lors de la déformation de l'une au moins des deux branches longues 102 et 103, il est prévu, en outre, que le parallélogramme que forme la plaque d'ostéosynthèse 100 présente un angle aigu α compris entre 50 et 80°, et de préférence sensiblement égal à 65°.

De cette façon on comprend (voir figure 3) que l'angle complémentaire β est compris entre 10 et 40°, de préférence sensiblement égal à 25°.

On comprend que la plaque d'ostéosynthèse 100 selon la présente invention permet de réaliser de manière fiable et tout à fait précise la fixation solidaire ou ostéosynthèse entre deux fragments osseux avec une possibilité de réglage de la position relative entre les deux fragments osseux. Ainsi, on évite la pose successive de plusieurs plaques qui entraîne la nécessité d'ettectuer plusieurs trous situés près les uns des autres et susceptibles de fragiliser le maxillaire.

En conséquence, la plaque d'ostéosynthèse selon la présente invention permet au chirurgien de réaliser un positionnement très précis entre les deux fragments osseux permettant de tenir compte de tous les paramètres anatomiques et physiologiques spécifiques au patient traité. En particulier, le réglage ultime de la position de la plaque, qui est réalisé par déformation de la plaque, tient compte de la remise naturelle du condyle dans son logement anatomique (glène) : la position du condyle au sein de l'articulation temporo-mandibulaire est en effet impossible à connaître lors de la pose de la plaque (position intermédiaire précitée).

## Revendications

1. Plaque d'ostéosynthèse (100), munie de quatre branches (101, 102, 103, 104) reliées entre elles deux à deux au niveau de quatre sommets (110) percés chacun par un alésage (112) destiné à coopérer avec une vis de fixation, au moins l'une des branches constituant une branche de réglage susceptible d'être déformée de manière à modifier l'écart entre les deux sommets (110) situés aux extrémités de ladite branche de réglage, **caracterisée en ce que** la largeur (L) de la ou les branches de réglage est inférieure au diamètre (D) desdits alésages (112), **en ce que** ladite plaque présente la forme d'un parallélogramme, et **en ce que** ledit parallélogramme présente un angle (α) au sommet aigu compris entre 50 degrés et 80 degrés.

2. Plaque d'ostéosynthèse selon la revendication 1, **caractérisée en ce que** la largeur (I) de ladite branche de réglage est comprise entre 20% et 60% du diamètre (D) desdits alésages (112).

3. Plaque d'ostéosynthèse selon la revendication 2, **caractérisée en ce que** largeur (I) de ladite branche de réglage est comprise entre 35 et 45% du diamètre (D) desdits alésages (112).

4. Plaque d'ostéosynthèse selon la revendication 3, **caractérisée en ce que** la largeur (I) de ladite branche de réglage est de l'ordre de 40% du diamètre (D) desdits alésages (112).

5. Plaque d'ostéosynthèse selon l'une quelconque des revendications précédentes, **caractérisée en ce qu'**elle présente une épaisseur (e) sensiblement égale à la largeur (I) de ladite branche de réglage.

6. Plaque d'ostéosynthèse selon l'une quelconque des revendications précédentes, **caractérisée en ce qu'**elle comporte deux branches longues (101, 103) et deux branches courtes (102, 104), et **en ce que** au moins l'une des deux branches longues (101, 103) présente une largeur (I) inférieure au diamètre (D) desdits alésages (112) afin de constituer ladite branche de réglage.

7. Plaque d'ostéosynthèse selon la revendication 1, **caractérisée en ce que** ledit angle (α) est sensiblement égal à 65 degrés.

8. Plaque d'ostéosynthèse selon l'une quelconque des revendications précédentes, **caractérisée en ce que** lesdits alésages (112) présentent une fraisure (114, 116) sur les deux faces de ladite plaque (100).

## Patentansprüche

1. Osteosyntheseplatte (100), die mit vier Schenkeln (101, 102, 103, 104) versehen ist, die untereinander paarweise auf der Ebene von vier Scheitelpunkten (110) verbunden sind, die mit jeweils einer Bohrung (112) versehenen sind, welche dazu vorgesehen ist, mit einer Fixierungsschraube zusammenzuwirken, wobei wenigstens einer der Schenkel einen Einstellschenkel bildet, der geeignet ist, derart deformiert zu werden, daß der Abstand zwischen den beiden Scheitelpunkten (110) modifiziert wird, die auf den Enden des Einstellschenkels liegen,
**dadurch gekennzeichnet,**
**daß** die Breite (I) des oder der Einstellschenkel kleiner ist als der Durchmesser (D) der Bohrungen (112),
**daß** die Platte die Form eines Parallelogramms aufweist und
**daß** das Parallelogramm einen Winkel (α) am spitzen Ende zwischen 50 Grad und 80 Grad aufweist.

2. Osteosyntheseplatte nach Anspruch 1, **dadurch gekennzeichnet, daß** die Breite (I) des Einstellschenkels zwischen 20 % und 60 % des Durchmessers (D) der Bohrung (112) liegt.

3. Osteosyntheseplatte nach Anspruch 2, **dadurch gekennzeichnet, daß** die Länge (I) des Einstellschenkels zwischen 35 % und 45 % des Durchmessers (D) der Bohrungen (112) liegt.

4. Osteosyntheseplatte nach Anspruch 3, **dadurch gekennzeichnet, daß** die Breite (I) des Einstellschenkels in der Größenordnung von 40 % des Durchmessers (D) der Bohrungen (112) ist.

5. Osteosyntheseplatte nach einem der vorhergehenden Ansprüche, **dadurch gekennzeichnet, daß** sie eine Dicke (e) aufweist, die im wesentlichen gleich der Breite (I) des Einstellschenkels ist.

6. Osteosyntheseplatte nach einem der vorhergehenden Ansprüche, **dadurch gekennzeichnet, daß** sie zwei lange Schenkel (101, 103) und zwei kurze Schenkel (102, 104) umfaßt, und **dadurch**, und daß wenigstens einer der beiden langen Schenkel (101, 103) eine Breite (I) kleiner als der Durchmesser (D) der Bohrungen (112) aufweist, um den Einstellschenkel zu bilden.

7. Osteosyntheseplatte nach Anspruch 1, **dadurch gekennzeichnet, daß** der Winkel (α) im wesentlichen gleich 65 Grad ist.

8. Osteosyntheseplatte nach einem der vorhergehenden Ansprüche, **dadurch gekennzeichnet, daß** die Bohrungen (112) eine Ausfräsung (114, 116) an den beiden Seiten der Platte (100) aufweisen.

## Claims

1. Osteosynthesis plate (100) provided with four limbs (101, 102, 103, 104) connected together in pairs at the level of their four vertices (110), each having a bore (112) through it intended to co-operate with a fixing screw, at least one of the limbs forming an adjustment limb able to be deformed so as to modify the spacing between the two vertices (110) situated at the ends of the said adjustment limb, **characterised by** the fact that the width (1) of the adjustment limb or limbs is less than the diameter (D) of the said bores (112), by the fact that the said plate has the form of a parallelogram, and by the fact that the said parallelogram has an angle (α) at the acute vertex of between 50 degrees and 80 degrees.

2. Osteosynthesis plate as described in claim 1, **characterised by** the fact that the width (1) of the said adjustment limb is between 20% and 60% of the diameter (D) of the said bores (112).

3. Osteosynthesis plate as described in claim 2, **characterised by** the fact that the width (1) of the said adjustment limb is between 35% and 45% of the diameter (D) of the said bores (112).

4. Osteosynthesis plate as described in claim 3, **characterised by** the fact that the width (1) of the said adjustment limb is of the order of 40% of the diameter (D) of the said bores (112).

5. Osteosynthesis plate as described in any one of the preceding claims, **characterised by** the fact that it has a thickness (e) substantially equal to the width (1) of the said adjustment limb.

6. Osteosynthesis plate as described in any one of the preceding claims, **characterised by** the fact that it includes two long limbs (101, 103) and two short limbs (102, 104), and by the fact that at least one of the two long limbs (101, 103) has a width (1) less than the diameter (D) of the said bores (112) in order to form the said adjustment limb.

7. Osteosynthesis plate as described in claim 1, **characterised by** the fact that the said angle (α) is substantially equal to 65 degrees.

8. Osteosynthesis plate as described in any one of the preceding claims, **characterised by** the fact that the said bores (112) are countersunk (114, 116) on the two faces of the said plate (100).
